# EUROPEAN PATENT APPLICATION

(11) **EP 4 692 084 A1**
(43) Date of publication of application: **11.02.2026**
(21) Application number: 24192778.9
(22) Date of filing: 05.08.2024
(51) Int. Cl.: C07D 451/06

(54) **NOVEL DERIVATIVES OF N-SUBSTITUTED NORTROPINONE, THEIR PRODUCTION AND THEIR USE**

(71) Applicant: Siegfried AG, 4800 Zofingen (CH)
(72) Inventor: BURGOS, Alain, 69230 Saint Genis Laval (FR)
(74) Representative: Isarpatent

(57) **Abstract**

The present invention relates to a compound of Formula I 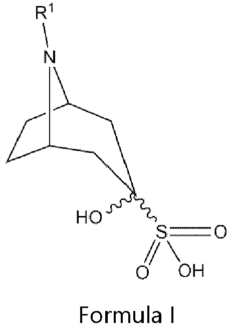 wherein R¹ is an optionally substituted alkyl, alkenyl or alkynyl residue with 1 to 10 carbon atoms, an optionally substituted aryl residue with 6 to 20 carbon atoms, an optionally substituted aralkyl residue with 7 to 21 carbon atoms, or an optionally substituted alkylaryl residue with 7 to 21 carbon atoms, preferably benzyl, a method of its production, and its use as intermediate in chemical syntheses, particularly of various active pharmaceutical actives.

## Description

The present invention relates to a compound of Formula I 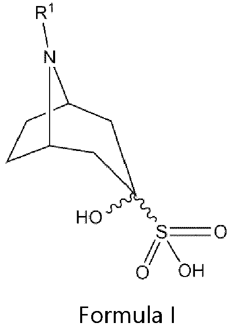 wherein R¹ is an optionally substituted alkyl, alkenyl or alkynyl residue with 1 to 10 carbon atoms, an optionally substituted aryl residue with 6 to 20 carbon atoms, an optionally substituted aralkyl residue with 7 to 21 carbon atoms, or an optionally substituted alkylaryl residue with 7 to 21 carbon atoms, preferably benzyl, a method of its production, and its use as intermediate in chemical syntheses, particularly of various active pharmaceutical ingredients / pharmaceutical actives (APIs).

### State of the art

N-benzyl nortropinone (N-benzyl-8-azabicyclo 3.2.1 octan-3-one) HCl is a starting material for active pharmaceutical ingredients like Trospium or Zatosetron, being used to produce nortropinone and afterwards endo-nortropine. While the synthesis of N-benzyl nortropinone base is fairly well described in the scientific literature, isolation of the HCl salt is a challenge. Filtration is difficult, and in acidic mixture, a significant amount of black tar is produced which contaminates the product. A particular problem is the isolation of N-benzyl nortropinone HCl. Moreover, the obtained viscous suspension of N-benzyl nortropinone HCl generally turns dark in the strong acidic conditions required for the formation of the HCl salt. Consequently, filtration rate can be extremely low, and depletion of process impurities and tar is not reproducible.

Another problem is scaling up, as the design of the process is not easy to scale due to problems with the reaction. Indeed, a strongly basic aqueous solution of acetonedicarboxylic acid and strongly acidic aqueous solution of benzylamine and dimethoxy-THF are usually co-added, maintaining the pH at 3.5, leading to the above problems.

A much simpler process is disclosed in Organic Process Research & Development 1997, 1, 198-210 to produce nortropinone base. An acidic solution of succinaldehyde made in situ from dimethoxy-THF is added to a solution of acetonedicarboxylic acid and benzylamine in a sodium acetate buffer solution at pH=5. However, the isolation of N-benzyl nortropinone HCl remains a challenge.

### Summary of the invention

The problem to be solved by the invention is thus to provide a simple and more reliable method of producing N-substituted-nortropinone that can avoid the disadvantages of formation of tar as well as the problems in scaling.

A first aspect of the invention relates to a compound of Formula I; 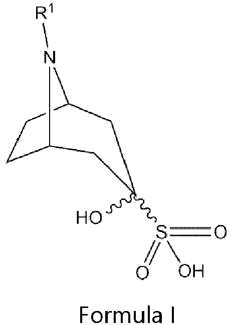 wherein R¹ is an optionally substituted alkyl, alkenyl or alkynyl residue with 1 to 10 carbon atoms, an optionally substituted aryl residue with 6 to 20 carbon atoms, an optionally substituted aralkyl residue with 7 to 21 carbon atoms, or an optionally substituted alkylaryl residue with 7 to 21 carbon atoms, preferably benzyl.

Furthermore disclosed in a second aspect is a method of preparing a compound of Formula I, comprising reacting a compound of Formula II, or a salt and/or an adduct thereof, with a sulfite source 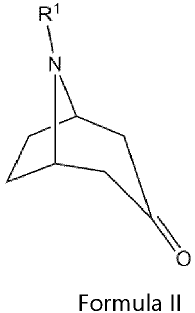 wherein R¹ is as described above.

A third aspect relates to a method of producing a compound of Formula II, as above, comprising reacting a compound of Formula I, as above, with a base.

Also disclosed is the use of a compound of Formula I, as above, as an intermediate in the production of a pharmaceutical active chosen from Trospium Chloride, Zatosetron, Retapamulin or Maraviroc.

Additionally, the present invention relates to a method of producing Trospium Chloride, Zatosetron, Retapamulin or Maraviroc, comprising:
preparing a compound of Formula II, or a salt and/or an adduct thereof, preferably a solution comprising an acid adduct of a compound of Formula II, further preferably an HCl adduct; reacting the compound of Formula II, or a salt and/or an adduct thereof, preferably the acid adduct, further preferably HCl adduct, of the compound of Formula II, with a sulfite source, particularly a sulfite salt, a disulfite salt and/or a hydrogensulfite salt, preferably in the presence of an acid, further preferably HCl, to prepare a compound of Formula I; optionally isolating the compound of Formula I; reacting the compound of Formula I with a base to produce a compound of Formula II; and reacting the compound of Formula II to Trospium Chloride, Zatosetron, Retapamulin or Maraviroc.

The invention also relates to a method of producing a compound of Formula II, comprising preparing a solution comprising an acid adduct of a compound of Formula II, preferably an HCl adduct; reacting the acid adduct, preferably HCl adduct, of the compound of Formula II, with a sulfite source, particularly a sulfite salt, a disulfite salt and/or a hydrogensulfite salt, in the presence of an acid, preferably HCl; optionally isolating the compound of Formula I; and reacting the compound of Formula I with a base to produce the compound of Formula II. Further aspects and embodiments of the invention are disclosed in the dependent claims and can be taken from the following description, figures and examples, without being limited thereto.

### Figures

The enclosed drawings should illustrate embodiments of the present invention and convey a further understanding thereof. In connection with the description, they serve as explanation of concepts and principles of the invention. Other embodiments and many of the stated advantages can be derived in relation to the drawings. The elements of the drawings are not necessarily to scale towards each other. Identical, functionally equivalent and acting equal features and components are denoted in the figures of the drawings with the same reference numbers, unless noted otherwise.
Figure 1 shows an ¹H-NMR spectrum of N-benzyl nortropinone bisulfite produced in the examples of the present invention measured in TFA-d (300 MHz).
Figure 2 shows a HPLC (Kinetex 150 x 3 mm ; 2,6 µm, 40 °C) spectrum of N-benzyl nortropinone bisulfite produced in the examples of the present invention.

### Detailed description of the present invention

### Definitions

It is noted that in this application a salt of an amine with an acid is also called an "acid adduct" or "adduct".

Unless defined otherwise, technical and scientific terms used herein have the same meaning as commonly understood by one of ordinary skill in the art to which this invention belongs. Amounts within the present invention are given in wt.%, unless stated otherwise or clear from context.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

As used herein, the terms "comprises", "comprising", "includes", "including", "has". "having" or any other variation thereof, are intended to cover a non-exclusive inclusion. For example, a process, method, article, or apparatus that comprises a list of elements is not necessarily limited to only those elements, but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In a first aspect, the present invention relates to a compound of Formula I; 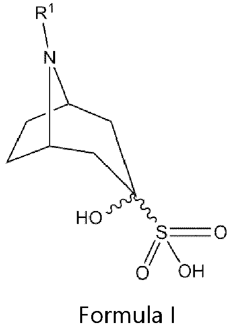 wherein R¹ is an optionally substituted alkyl, alkenyl or alkynyl residue with 1 to 10 carbon atoms, an optionally substituted aryl residue with 6 to 20 carbon atoms, an optionally substituted aralkyl residue with 7 to 21 carbon atoms, or an optionally substituted alkylaryl residue with 7 to 21 carbon atoms, preferably benzyl. The optional substituent is not particularly restricted, but is preferably chosen from the halogens F, Cl, Br, I, and/or a protective group. Preferably, residue R¹ is unsubstituted, i.e. chosen from an alkyl, alkenyl or alkynyl residue with 1 to 10 carbon atoms, an aryl residue with 6 to 20 carbon atoms, an aralkyl residue with 7 to 21 carbon atoms, or an alkylaryl residue with 7 to 21 carbon atoms, preferably benzyl. Further, preferably, residue R¹ is chosen from an alkyl residue with 1 to 6 carbon atoms, an aryl residue with 6 to 10 carbon atoms, an aralkyl residue with 7 to 11 carbon atoms, or an alkylaryl residue with 7 to 11 carbon atoms, preferably benzyl (i.e. -CH₂-C₆H₅).

According to certain embodiments, the compound of Formula I is a mixture of the compounds of Formula la and Formula Ib, with R¹ as defined above, the former with the OH group in endo position, i.e. located closest to the longest bridge of the bridged ring system, and the latter with the OH group in exo position. The mixing ratio is not restricted.

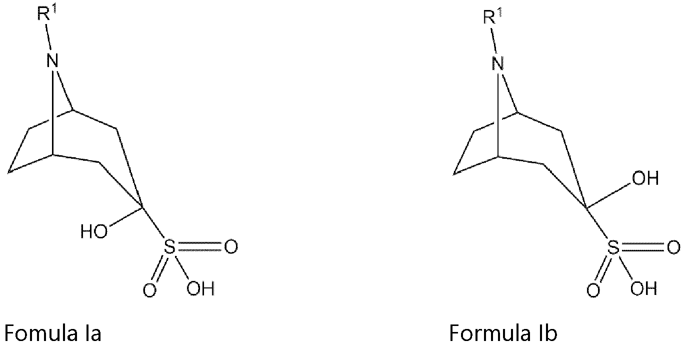

According to certain embodiments, the compound of Formula I is a compound of Formula la. According to certain embodiments, the compound of Formula I is a compound of Formula lb. According to certain embodiments, the compound of Formula I is a compound of Formula Ia'. According to certain embodiments, the compound of Formula I is a compound of Formula Ib'. According to certain embodiments, the compound of Formula I is a compound of Formula I', i.e. a mixture of the compounds of Formula Ia' and Formula Ib'. The mixing ratio is not restricted.

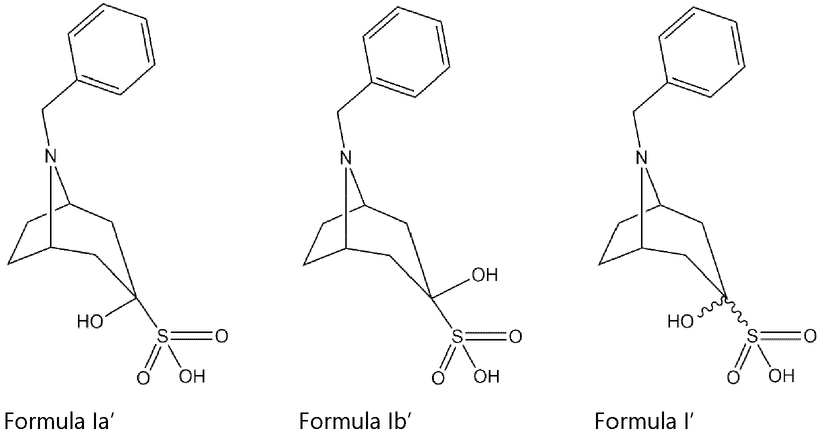

It has been found that the compound of Formula I, respectively the compounds of Formula la, Formula Ib, Formula I', Formula Ia', and Formula Ib', surprisingly is, respectively are, very stable and can be produced in a simple manner without a huge amount of side reactions, particularly without side products that are difficult to remove.

A second aspect of the invention relates to a method of preparing a compound of Formula I, comprising reacting a compound of Formula II, or a salt and/or an adduct thereof, with a sulfite source; 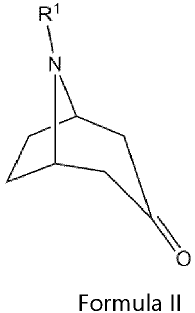 wherein R¹ is as defined above.

In the method, the sulfite source is not particularly restricted. Preferably, the sulfite source is a sulfite salt, a disulfite salt and/or a hydrogensulfite salt. The compound of Formula II, or salt and/or adduct thereof, is not particularly restricted and can be obtained by any suitable method. Preferably, the adduct is an acid adduct, and particularly preferably an adduct, particularly acid adduct of the compound of Formula II is used for the reaction.

According to certain embodiments, the reaction is performed in the presence of an acid. The acid therein is not particularly restricted and is preferably HCl.

According to certain embodiments, the compound of Formula II, or a salt thereof, is reacted with an acid prior to the reaction with the sulfite source. The acid is not particularly restricted and is preferably HCl.

According to certain embodiments, the method of the second aspect comprises preparing a solution comprising an acid adduct of a compound of Formula II, preferably an HCl adduct; reacting the acid adduct, preferably HCl adduct, of the compound of Formula II, with a sulfite source, which may be a sulfite salt, a disulfite salt and/or a hydrogensulfite salt, in the presence of an acid, preferably HCl; and optionally isolating the compound of Formula I.

Particularly, the compound of Formula Ia', the compound of Formula Ib', or the compound of Formula I', can be produced from a compound of Formula II' or a salt and/or adduct thereof.

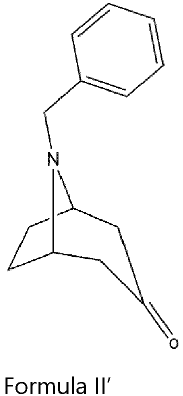

The method of preparing the acid adduct of the compound of Formula II, preferably of the compound of Formula II'; is not particularly restricted, and can be done e.g. in solution as is known in the prior art, e.g. from the production of N-benzyl nortropinone. An exemplary method of preparing an exemplary compound of Formula II, with the subsequent formation of an HCl adduct thereof, as known in literature, is shown in the following scheme, with benzylamine used for forming a benzyl group as R¹:

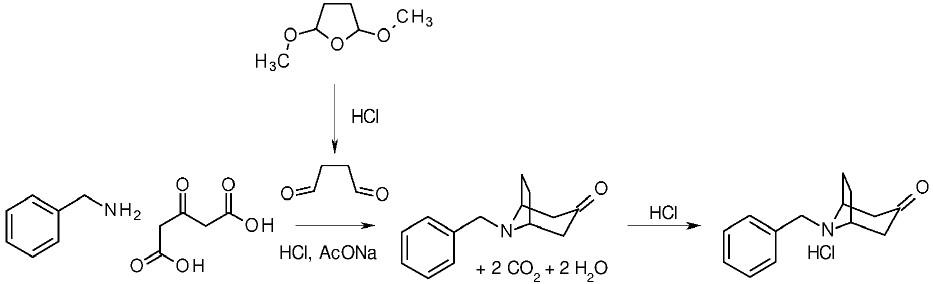

Also, the reaction of the acid adduct with the sulfite source, preferably a sulfite salt, a disulfite salt and/or a hydrogensulfite salt, in the presence of an acid is not restricted, and the sulfite salt, disulfite salt and/or hydrogensulfite salt can e.g. be just added to a solution in which the acid adduct of the compound of the compound of Formula II was prepared.

In the method, the sulfite source, preferably the sulfite salt, disulfite salt (metabisulfite) and/or hydrogensulfite (bisulfite) salt, is not particularly restricted, but is preferably an alkali metal salt, further preferably a sodium and/or potassium salt, i.e. sodium sulfite, sodium disulfite, sodium hydrogensulfite, potassium sulfite, potassium disulfite and/or potassium hydrogensulfite. Further preferable is the use of a sodium salt, particularly sodium disulfite and/or sodium hydrogensulfite, for ease of reaction. If the disulfite salt is used, it is preferred that the reaction proceeds in the presence of water. According to certain embodiments, the reaction is carried out in the presence of water. It is even not excluded that the reaction is carried out in water as sole solvent. While water can be contained as a solvent, it is not excluded that further solvents are included, e.g. from a prior production of the compound of Formula II, respectively the compound of Formula II'.

The reaction temperature is not particularly restricted. According to certain embodiments, the reaction is carried out at a temperature of 20°C - 80°C, preferably 30°C - 70°C, further preferably 45°C - 55°C. At such temperature, preparing the compound of Formula I is further improved.

After the reaction, the reaction liquid can be cooled to room temperature and the compound of Formula I can be isolated, e.g. by filtration, and optionally be washed, e.g. with water, and/or dried.

As disclosed, the reaction is preferably carried out in the presence of an acid that is not particularly restricted, but preferably is a stong, non oxidizing acid like HCl or H₂SO₄, preferably HCl, further preferably aqueous HCl. While the pH of the reaction is not particularly restricted, the reaction with the sulfite source, particularly sulfite salt, disulfite salt and/or hydrogensulfite salt, is preferably carried out at a pH in aqueous solution between and including 2.5 and 5.0, further preferably between and including 2.8 and 3.2. At such a pH, the formation of the compound of Formula I is further improved. In order to achieve such preferable pH, a buffer substance like citric acid can be added to the reaction mixture. A further improved yield can be obtained using a suitable buffer substance.

With the method of the second aspect, the compound of Formula I can be produced, which is a superior alternative in the production of APIs based on the compound of Formula II or base products thereof without intermediate formation of a compound of Formula I, e.g. Trospium Chloride, Zatosetron, Retapamulin or Maraviroc.

The method of this second aspect can, for example, comprise a step 1 of preparing a solution comprising an acid adduct of a compound of Formula II, a step 2 of reacting the acid adduct of the compound of Formula II with a sulfite source, particularly a sulfite salt, a disulfite salt and/or a hydrogensulfite salt, in the presence of an acid following after such step, immediately or after some intermediate steps, and an optional step 3 of isolating the compound of Formula I, which can be carried out directly after step 2 or after some further intermediate steps.

A third aspect of the invention relates to a method of producing a compound of Formula II, comprising reacting a compound of Formula I with a base. 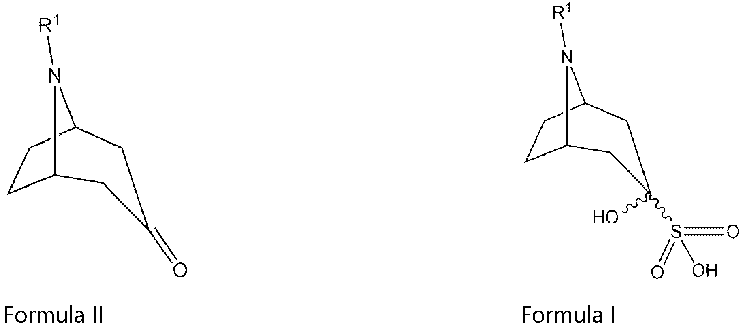 wherein R¹ is as defined above. In the method, the residue R¹ is the same as in the compound of the first aspect, and reference is made thereto for the sake of brevity.

In the method of the third aspect, the base is not particularly restricted. Any base can be used, but preferably the base is an inorganic base, e.g. NaOH, KOH, etc., for easier separation of the compound of Formula II afterwards. Also, the reaction conditions are not particularly restricted.

A fourth aspect relates to the use of a compound of Formula I as an intermediate in the production of a pharmaceutical active chosen from Trospium Chloride, Zatosetron, Retapamulin or Maraviroc.

In a fifth aspect, a method of producing Trospium Chloride, Zatosetron, Retapamulin or Maraviroc is disclosed, comprising: preparing a compound of Formula II, or a salt and/or an adduct thereof, preferably a solution comprising an acid adduct of a compound of Formula II, further preferably an HCl adduct; reacting the compound of Formula II, or a salt and/or an adduct thereof, preferably the acid adduct, further preferably HCl adduct, of the compound of Formula II, with a sulfite source, particularly a sulfite salt, a disulfite salt and/or a hydrogensulfite salt, preferably in the presence of an acid, further preferably HCl, to prepare a compound of Formula I; optionally isolating the compound of Formula I; reacting the compound of Formula I with a base to produce a compound of Formula II; and reacting the compound of Formula II to Trospium Chloride, Zatosetron, Retapamulin or Maraviroc; 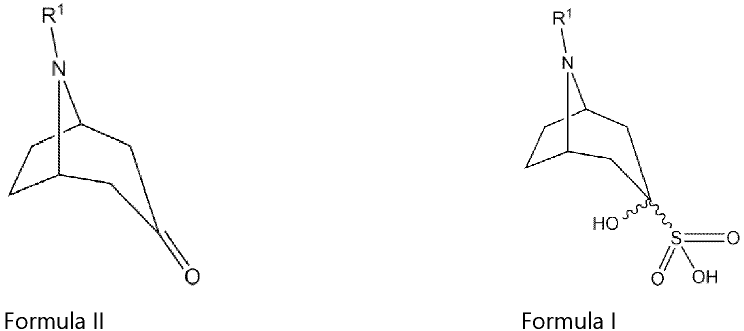 wherein R¹ is as defined above.

In the method of the fifth aspect, the steps of preparing a compound of Formula II, or a salt and/or an adduct thereof, preferably a solution comprising an acid adduct of a compound of Formula II, further preferably an HCl adduct; reacting the compound of Formula II, or a salt and/or an adduct thereof, preferably the acid adduct, further preferably HCl adduct, of the compound of Formula II, with a sulfite source, particularly a sulfite salt, a disulfite salt and/or a hydrogensulfite salt, preferably in the presence of an acid, further preferably HCl, to prepare a compound of Formula I; optionally isolating the compound of Formula I; reacting the compound of Formula I with a base to produce a compound of Formula II are the same as in the method of the second and third aspect, to which herein is referred to.

The step of reacting the compound of Formula II to Trospium Chloride, Zatosetron, Retapamulin or Maraviroc can be carried out as is known from the synthesis of these compounds, e.g. via nortropinone and endo-nortropine as intermediates.

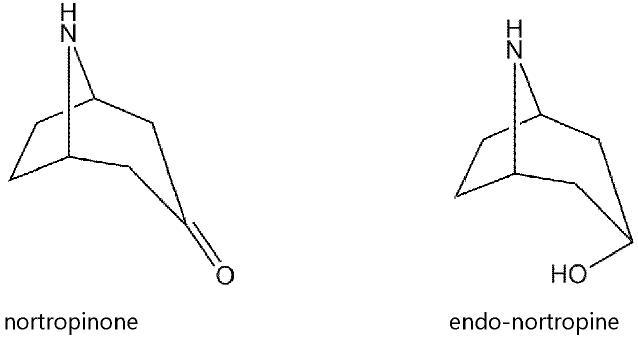

In the method, again the residue R¹ is as defined above.

A method of the fifth aspect comprises, for example, steps 1, 2 and 3 as in the second aspect, a step 4 of reacting the compound of Formula I with a base to produce a compound of Formula II, and a step 5 a step of reacting the compound of Formula II to Trospium Chloride, Zatosetron, Retapamulin or Maraviroc.

A sixth aspect of the invention relates to a method of producing a compound of Formula II, comprising: preparing a solution comprising an acid adduct of a compound of Formula II, preferably an HCl adduct; reacting the acid adduct, preferably HCl adduct, of the compound of Formula II, with a sulfite source, particularly a sulfite salt, a disulfite salt and/or a hydrogensulfite salt, in the presence of an acid, preferably HCl; optionally isolating the compound of Formula I; and reacting the compound of Formula I with a base to produce the compound of Formula II; 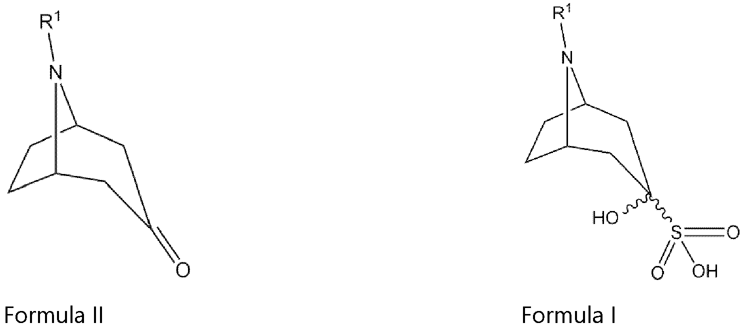 wherein R¹ is as defined above.

An exemplary method of the sixth aspect comprises steps 1 to 4 corresponding to those of the fifth aspect.

The above embodiments can be combined arbitrarily, if appropriate. Further possible embodiments and implementations of the invention comprise also combinations of features not explicitly mentioned in the foregoing or in the following with regard to the Examples of the invention. Particularly, a person skilled in the art will also add individual aspects as improvements or additions to the respective basic form of the invention.

### Examples

The present invention will now be described in detail with reference to several examples thereof. However, these examples are illustrative and do not limit the scope of the invention.

### Example 1.1: Preparation of 8-benzyl-8-azabicyclo[3.2.1]octan-3-ol-3-sulfonic acid hydrochloride

The process is based on the experimental procedure described in Organic Process Research & Development 1997, 1, 198-210. However, it is adapted to prepare the title compound, as follows.

In a first reaction vessel R1, 2,5-dimethoxytetrahydrofuran (41.4 g, 1.1 equivalents), water (45.5 g, 1.5 volumes), and 33 w/w% aqueous HCl (3.7 g, 0.12 equivalents) were charged and heated to 75°C. The mixture was maintained at 75°C for not less than 1 hour. Thereafter, the mixture is cooled to room temperature (20-25°C) to obtain a solution containing succinaldehyde quantitatively for later use.

In a second reaction vessel R2, water (150 g, 5 volumes), sodium acetate (57.4 g, 2.5 equivalents), benzylamine (30 g, 1 equivalent), and 33 w/w% aqueous HCl (0.48 equivalents) were charged and the mixture cooled to 10°C. Acetonedicarboxylic acid (44.5 g, 1.09 equivalents) is charged, and then the solution containing succinaldehyde from the first reaction vessel is added in the course of one hour and thirty minutes at a temperature below 15°C. After the addition, the reaction mixture is heated to 40°C over the course of 1 hour and the temperature is maintained for not less than 1 hour. The reaction mixture is cooled to room temperature and the pH is adjusted to pH 2.9 with 33 w/w% aqueous HCl (83 g, 2.66 equivalents). The reaction mixture is heated to 50°C, sodium metabisulfite (34.6 g, 0.65 equivalents) is charged, and the reaction mixture is cooled to room temperature. The precipitate is isolated by filtration from the crude reaction mixture in a Büchner funnel, and washed twice with 3 volumes (90 g) of water. The solid is dried at 40°C under vacuum to constant weight. The product was isolated with a yield of 48%.

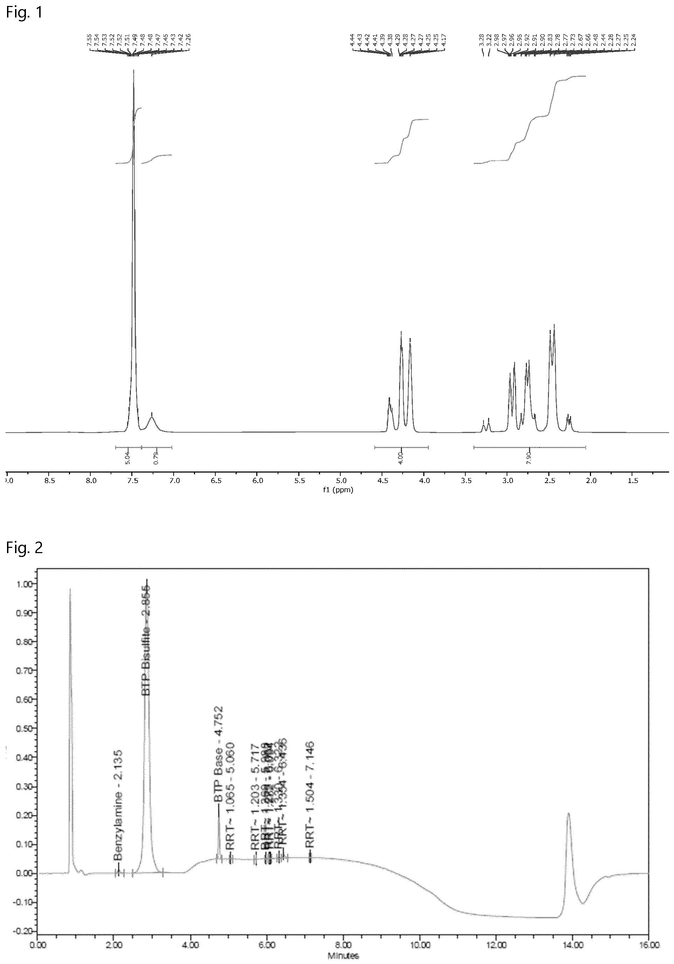

A ¹H NMR spectrum of the obtained compound was taken in TFA-d (300 MHz), and the resulting spectrum is shown in Figure 1, with the peaks being assigned as follows and confirming the structure of Formula I', i.e. having obtained a mixture of the compounds of Formula Ia' and Ib': aromatic δ 7.68 - 7.38 (m, 5H), alcohol δ 7.26 (brs, 1H), Benzylic and CH alpha hydrogen δ 4.57 - 3.95 (m, 4H), bridge and CH₂ alpha alcohol (endo + exo) δ 3.39 - 2.06 (m, 8H) ppm. The amino function is broad and not visible but is confirmed in the following elemental analysis and mass spectroscopy.

Elemental analysis gave the following: Carbon 56.3% (56.55%); Hydrogen 6.45% (6.44%); Nitrogen 4.69% (4.71%); Oxygen 21.71% '(21.52%); Sulfur 10.67% (10.78%).

Mass spectrometry using electrospray negative ionization ("ESI") resulted in peaks at 296.0956 (m/z) for the obtained zwitter ion, a peak at 593.2111 for a dimer minus the mass of HO₃S⁻ (80.9562), confirming formation of the zwitter ion as mixture further:

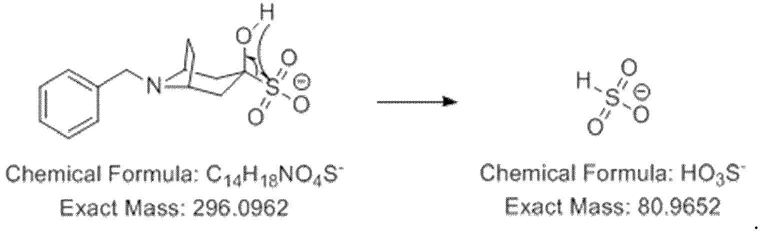

ESI positive ionization experiments confirmed the following peaks when spraying the zwitter ion:

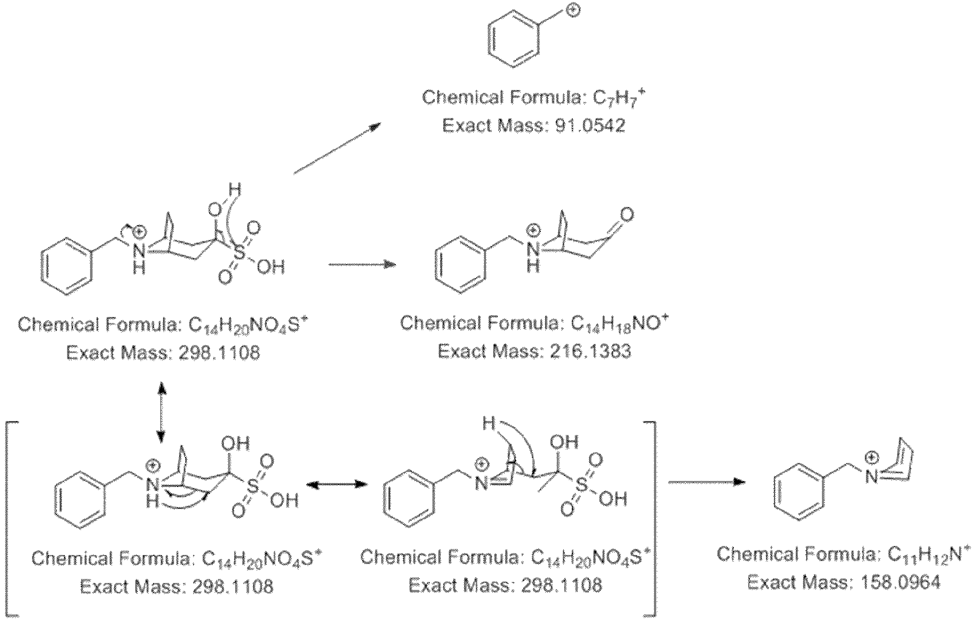

Analysis with HPLC (Kinetex 150 x 3 mm ; 2,6 µm, 40 °C) with the scheme in the following table and detection at 210 nm gave the spectrum in Figure 2.

| Flow 0.8ml/mn | A = 95/5 H2O/ACN +0,1% TFA | B = 5/95 H2O/ACN +0,1% TFA |
|---|---|---|
| 0.00 mn | 100 | 0 |
| 2.00 mn | 100 | 0 |
| 9.00 mn | 0 | 100 |
| 12.00 mn | 0 | 100 |
| 12.01 mn | 100 | 0 |
| 16.00 mn | 100 | 0 |

The retention times were annotated as follows: Benzylamine 2.13 min; N-Benzyltropinone bisulfite 2.86 min; N-Benzyltropinone 4.75min.

### Example 1.2:

Example 1.2 was carried out as example 1.1, except that the amount of 2,5-dimethoxytetrahydrofuran in reaction vessel R1 was reduced to 1.0 equivalents. The product was isolated with a yield of 54%.

### Example 1.3:

Example 1.3 was carried out as example 1.1, except that the scale was only half, and the pH during the addition of the succinaldehyde solution was monitored. Succinaldehyde was added to a solution of citric acid (0.6 eqivalents) and benzylamine (1 equivalent) at 20 °C. The pH dropped from 4.9 to 3.3. In order to minimize accumulation of the product (sulfonic acid derivative), the addition of sodium metabisulfite was run at 50°C, and the product was isolated with a yield of 67%.

### Example 2:

Formation of the bisulfite compound was tested by direct reaction of sodium metabisulfite with N-benzyl nortropinone HCl at a scale of 10 g at pH 3 in water, with the reaction conditions otherwise as above. The precipitation occurs immediately after the addition of NazSzOs., and the compound is obtained with a yield of 95.7%.

### Example 3:

In order to test the performance of the obtained compound N-benzyl-nortropinone bisulfite, the N-benzyl nortropinone base was formed from it, as follows, on a scale of 5 g with the bisulfite compound obtained from the 10 g scale experiment in Example 2. Two volumes water, NaOH 30 w/w% (2 equivalents), methyl tert-butyl ether (4 volumes) and N-benzyl nortropinone bisulfite (1 equivalent) were charged and stirred until complete dissolution is observed. The phases are split, and the lower aqueous phase is discarded. The organic phase is washed twice with water (1 volume) and evaporated to dryness. The hydrolysis is fast. N-benzyl nortropinone was isolated with 93% yield.

### Example 4:

In order to demonstrate that the use of the bisulfite compound has no negative impact on the downstream chemistry, nortropine base was produced from N-benzyl nortropinone bisulfite. The experiment is carried out on a scale of 47 g with the bisulfite compound obtained from the 50 g scale experiment in Example 2.

2.4 volumes water, NaOH 30 w/w% (2 equivalents), toluene (4.7 volumes) and N-benzyl nortropinone bisulfite (1 equivalent) were charged and stirred until complete dissolution is observed. The phases are split, and the lower aqueous phase is discarded. The organic phase is washed twice with brine 20 w/w% (1.2 volumes) and evaporated to dryness. 1.7 volumes isopropanol is charged, and again evaporation to dryness is carried out. 2.9 volumes of isopropanol (2.9 volumes), water (0.4 volumes), HCl 33 w/w% (1 equivalent) and 5% Pd/C (5%Pd/C Evonik, 700832; 0.12mol%, 0.02parts) are charged, and the atmosphere is replaced by nitrogen with 3 nitrogen purges at 5 bar. The nitrogen atmosphere is replaced by hydrogen with 5 hydrogen purges at 5 bar. The reaction mixture is stirred at 1000 rpm and heated to 45°C (internal temperature), and the reaction conditions are maintained until hydrogen uptake ceases. A sample is pulled for analysis. The mixture is cooled to 20°C, NaOH 50 w/w% (1.1 equivalents) is charged, and stirring is carried out for 30 minutes at 20°C. Celite (0.05 parts) is charged and the suspension is filtered. The solid is washed with a mixture of IPA (isopropyl alcohol) and water (respectively 0.3 and 0.05 volumes), and the pH of the lower aqueous layer (12.5≤pH≤ 13) is checked. The phases are split and the lower aqueous layer is discarded. Raney Nickel (0.2 parts) is charged and the atmosphere replaced by nitrogen with 3 nitrogen purges at 5 bar, followed by replacing the nitrogen atmosphere by hydrogen with 3 hydrogen purges at 5 bar. The mixture is stirred at 1000rpm and heated to 50°C (internal temperature), and the reaction conditions are maintained until hydrogen uptake ceases. A sample is pulled for analysis. Celite (0.02 parts) is charged and the suspension is filtered. The solid is washed with a mixture of IPA and water (respectively 0.4 and 0.1 volumes), and the mixture concentrated under reduce pressure to 0.8 volumes at 35°C. Toluene (3.7 volumes) is charged and an azeotropic distillation of water is performed under reduced pressure at 80°C, followed by cooling to 20°C. Alkaline charcoal (0.02 parts) is charged, and the mixture heated to 80°C (internal temperature) and held at this temperature for not less than 1 hour, followed by filtering the suspension at 80°C. The solid is washed with toluene (0.5 volumes), and concentration is carried out under reduce pressure to 1.3 volumes at 85°C. Again, toluene (0.5 volumes) is charged, and concentration is carried out under reduce pressure to 1.3 volumes at 85°C. A sample is pulled and the IPA content determined (<0.5 w/w% or <0.25 A%). The mixture is cooled to 0°C and the precipitate isolated. The solid is washed with toluene (0.4 volumes) and dried at 40°C under reduce pressure. The yield is 80%. The hydrogenolysis rate and the hydrogenation rate were identical to those obtained with N-benzyl nortropinone HCl in former experiments. The product before charcoal treatment is less colored.

### Example 5: Trospium chloride synthesis

In a first step, nortropine is formed as above. In a second step, dimethylformamide (DMF) (2 volumes) and 1,4-dichlorobutane (2 equivalents) are charged in a reaction vessel 1. The mixture is heated to 87°C (internal temperature). In a reaction vessel 2, DMF (5.4 volumes), 1,8-diazabicyclo(5.4.0)undec-7-ene (DBU) (1.5 equivalents) and nortropine (1 equivalent) are charged and the mixture is stirred until dissolution is observed. The solution in reaction vessel 2 is added into reaction vessel 1 over 1 hour at 87°C, and the mixture is maintained at 87°C for 1 hour, followed by cooling to 20°C. The precipitate is isolated, washed with DMF (2 volumes), washed with acetone (9 volumes), and dried at 30°C under reduce pressure. The yield is 88%.

In a third step, in a reaction vessel 1, DMF (3.8 volumes), 1'-carbonyldiimidazole (CDI) (1.5 equivalents) and benzylic acid (1.5 equivalents) are charged. In a reaction vessel 2, 1 equivalent of the compound from step 2, DMF (7.3 volumes) and DBU (0.5 equivalents) are charged, the mixture is heated to 80°C. The solution in reaction vessel 2 is transferred into reaction vessel 1 at 80°C. The mixture is maintained at 80° for 2 hours, then cooled to 0°C. The solid is washed with DMF (3.9 volumes), with acetone (8.6 volumes), and dried at 30°C under reduce pressure. The yield of trospium chloride corrected from the assay is 85%.

## Claims

1. A compound of Formula I 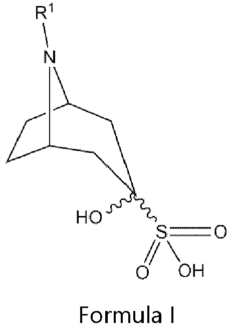 wherein R¹ is an optionally substituted alkyl, alkenyl or alkynyl residue with 1 to 10 carbon atoms, an optionally substituted aryl residue with 6 to 20 carbon atoms, an optionally substituted aralkyl residue with 7 to 21 carbon atoms, or an optionally substituted alkylaryl residue with 7 to 21 carbon atoms, preferably benzyl.

2. A method of preparing a compound of Formula I, comprising reacting a compound of Formula II, or a salt and/or adduct thereof, with a sulfite source; 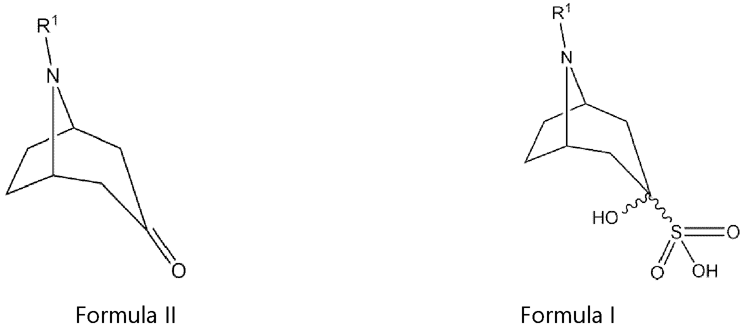 wherein R¹ is an optionally substituted alkyl, alkenyl or alkynyl residue with 1 to 10 carbon atoms, an optionally substituted aryl residue with 6 to 20 carbon atoms, an optionally substituted aralkyl residue with 7 to 21 carbon atoms, or an optionally substituted alkylaryl residue with 7 to 21 carbon atoms, preferably benzyl.

3. The method according to claim 2 wherein the reaction is performed in the presence of an acid.

4. The method according to claim 2 or 3 wherein the compound of Formula II, or a salt thereof, is reacted with an acid prior to the reaction with the sulfite source.

5. The method according to any one of claims 2 to 4, comprising preparing a solution comprising an acid adduct of a compound of Formula II, preferably an HCl adduct;
reacting the acid adduct, preferably HCl adduct, of the compound of Formula II, with the sulfite source, preferably a sulfite salt, a disulfite salt and/or a hydrogensulfite salt, in the presence of an acid, preferably HCl; and
optionally isolating the compound of Formula I.

6. The method of any one of claims 2 to 5, wherein the reaction with the sulfite source, particularly a sulfite salt, a disulfite salt and/or a hydrogensulfite salt, is carried out at a temperature of 20°C - 80°C, preferably 30°C - 70°C, further preferably 45°C - 55°C.

7. The method of any one of claims 2 to 6, wherein the reaction with the sulfite source, particularly a sulfite salt, disulfite salt and/or hydrogensulfite salt, is carried out in the presence of water at a pH between and including 2.5 and 5.0, preferably between and including 2.8 and 3.2.

8. A method of producing a compound of Formula II, comprising reacting a compound of Formula I with a base. 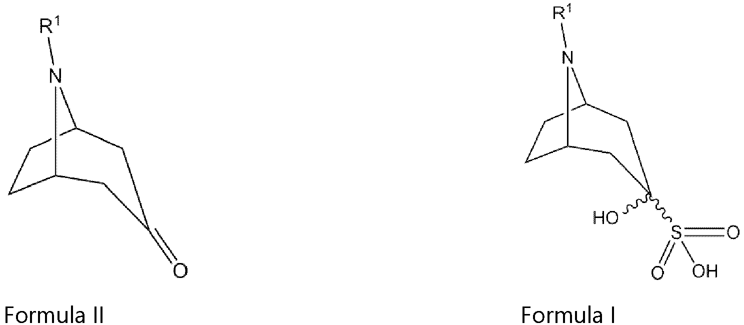 wherein R¹ is an optionally substituted alkyl, alkenyl or alkynyl residue with 1 to 10 carbon atoms, an optionally substituted aryl residue with 6 to 20 carbon atoms, an optionally substituted aralkyl residue with 7 to 21 carbon atoms, or an optionally substituted alkylaryl residue with 7 to 21 carbon atoms, preferably benzyl.

9. Use of a compound of Formula I as an intermediate in the production of a pharmaceutical active chosen from Trospium Chloride, Zatosetron, Retapamulin or Maraviroc 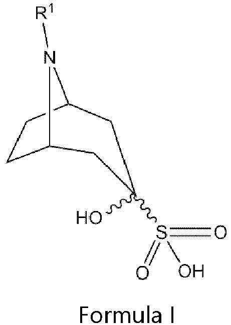 wherein R¹ is an optionally substituted alkyl, alkenyl or alkynyl residue with 1 to 10 carbon atoms, an optionally substituted aryl residue with 6 to 20 carbon atoms, an optionally substituted aralkyl residue with 7 to 21 carbon atoms, or an optionally substituted alkylaryl residue with 7 to 21 carbon atoms, preferably benzyl.

10. A method of producing Trospium Chloride, Zatosetron, Retapamulin or Maraviroc, comprising:
preparing a compound of Formula II, or a salt and/or an adduct thereof, preferably a solution comprising an acid adduct of a compound of Formula II, further preferably an HCl adduct;
reacting the compound of Formula II, or a salt and/or an adduct thereof, preferably the acid adduct, further preferably HCl adduct, of the compound of Formula II, with a sulfite source,
particularly a sulfite salt, a disulfite salt and/or a hydrogensulfite salt, preferably in the presence of an acid, further preferably HCl, to prepare a compound of Formula I;
optionally isolating the compound of Formula I;
reacting the compound of Formula I with a base to produce a compound of Formula II; and
reacting the compound of Formula II to Trospium Chloride, Zatosetron, Retapamulin or
Maraviroc; 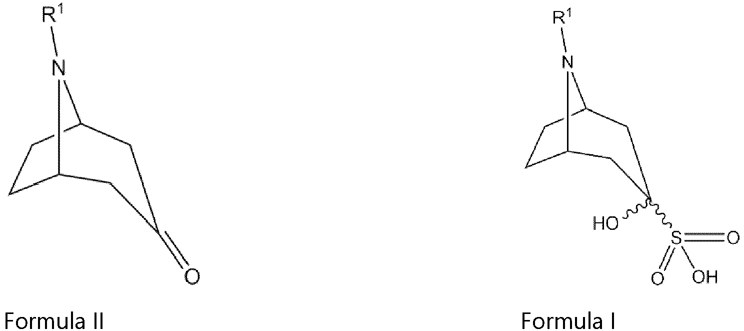
wherein R¹ is an optionally substituted alkyl, alkenyl or alkynyl residue with 1 to 10 carbon atoms, an optionally substituted aryl residue with 6 to 20 carbon atoms, an optionally substituted aralkyl residue with 7 to 21 carbon atoms, or an optionally substituted alkylaryl residue with 7 to 21 carbon atoms, preferably benzyl.

11. A method of producing a compound of Formula II, comprising:
preparing a solution comprising an acid adduct of a compound of Formula II, preferably an HCl adduct;
reacting the acid adduct, preferably HCl adduct, of the compound of Formula II, with a sulfite source, particularly a sulfite salt, a disulfite salt and/or a hydrogensulfite salt, in the presence of an acid, preferably HCl;
optionally isolating the compound of Formula I; and
reacting the compound of Formula I with a base to produce the compound of Formula II; 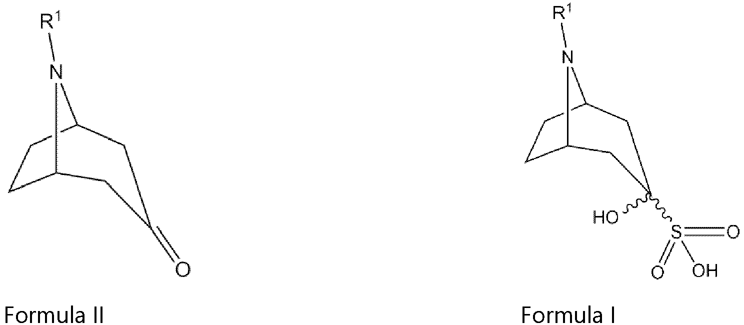
wherein R¹ is an optionally substituted alkyl, alkenyl or alkynyl residue with 1 to 10 carbon atoms, an optionally substituted aryl residue with 6 to 20 carbon atoms, an optionally substituted aralkyl residue with 7 to 21 carbon atoms, or an optionally substituted alkylaryl residue with 7 to 21 carbon atoms, preferably benzyl.
